# FASCICULE DE BREVET EUROPEEN

(11) **EP 0 910 657 B1**
(45) Date de publication et mention de la délivrance du brevet: **27.02.2013**
(21) Numéro de dépôt: 97930568.7
(22) Date de dépôt: 23.06.1997
(51) Int. Cl.: C12N 15/86, A61K 39/295, C12N 15/38, C12N 7/01, C07K 14/015, C07K 14/115, C07K 14/13, C07K 14/145, C07K 14/20, C07K 14/235

(54) **VACCIN VIVANT RECOMBINANT A BASE D'HERPESVIRUS CANIN, NOTAMMENT CONTRE LA MALADIE DE CARRE, LA RAGE OU LE VIRUS PARAINFLUENZA DE TYPE 2**
REKOMBINANTER LEBENDIMPFSTOFF BASIEREND AUF HUNDE-HERPESVIRUS, INSBESONDERE GEGEN STAUPE, TOLLWUT UND PARAINFLUENZA VIRUS TYP 2
CANINE HERPESVIRUS BASED RECOMBINANT LIVE VACCINE, IN PARTICULAR AGAINST CANINE DISTEMPER, RABIES OR THE PARAINFLUENZA 2 VIRUS

(30) Priorité: 27.06.1996 FR 9608242
(43) Date de publication de la demande: 28.04.1999
(73) Titulaire: MERIAL, 69007 Lyon (FR)
(72) Inventeur: AUDONNET, Jean-Christophe, F-69006 Lyon (FR); BAUDU, Philippe, F-69240 Craponne (FR)
(74) Mandataire: Cabinet Plasseraud
(86) Numéro de dépôt international: PCT/FR1997/001115
(87) Numéro de publication internationale: WO 1997/049825

(56) Documents cités:
- EP-A- 0 447 303
- WO-A-95/26751
- US-A- 4 213 965
- REMOND M ET AL: "Gene organization in the UL region and inverted repeats of the canine herpesvirus genome." JOURNAL OF GENERAL VIROLOGY, (1996 JAN) 77 ( PT 1) 37-48., XP000543180 cité dans la demande
- REMOND, M. ET AL.: "Sequence of the canine herpesvirus thymidine kinase gene: taxon-preferred amino acid residues in the alphaherpesviral thymidine kinases" VIRUS RES. (1995), 39(2-3), 341-54, XP000646056 cité dans la demande
- DATABASE EPODOC European Patent Office GAO YUN ET AL.: "Triple live vaccine and toxine vaccine for distemper, rabies and parvovirus and its preparing method" XP002027378 & CN 1 117 081 A (GAO YUN) 21 février 1996
- LIMBACH K J ET AL: "Nucleotide sequence of the genes encoding the canine herpesvirus gB, gC and gD homologues." JOURNAL OF GENERAL VIROLOGY, (1994 AUG) 75 ( PT 8) 2029-39., XP002027821 cité dans la demande

## Description

La présente invention a trait à des vaccins, de préférence pour chiens, produits à partir d'herpèsvirus canins recombinants, et aux méthodes d'obtention et de préparation de ces virus recombinants. La présente invention a plus particulièrement trait aux recombinants herpèsvirus canins comprenant une cassette d'expression pour un (ou des) gène(s) étranger(s).

L'herpèsvirose canine est causée par l'herpèsvirus canin (en anglais Canine HerpesVirus ou CHV). L'herpèsvirus canin (CHV) est classé dans la famille des *Alphaherpesvirinae.* Cet herpèsvirus est un pathogène majeur pour les chiots à la naissance. L'herpèsvirose canine se traduit principalement par une maladie hémorragique chez le chiot et par une maladie bénigne de l'appareil respiratoire supérieur chez les chiens adultes. Il n'existe actuellement pas de vaccins pour la protection des chiots contre l'herpèsvirose canine.

Par ailleurs, les chiens domestiques sont exposés à de nombreuses autres maladies, et la mise au point d'un vecteur vaccinal pouvant exprimer différents antigènes d'agents pathogènes canins permettrait de simplifier et d'améliorer l'efficacité des programmes de vaccination, en particulier des chiots dans les élevages. Parmi les agents pathogènes d'importance pour le chien, on peut citer le virus de la maladie de Carré, le virus de l'hépatite de Rubarth, le virus de la rage, le virus de la parvovirose canine, le coronavirus canin, le virus parainfluenza de type 2, *Bordetella bronchiseptica, Borrelia burgdorferi, Leptospira spp., Leishmania infantum.*

On connaît peu de choses sur le génome du virus CHV. L'organisation génomique de ce virus n'a été publiée que récemment (Rémond M. et al. J. Gen. Virol. 1996. 77. 37-48) et les gènes des trois glycoprotéines majeures gB, gC et gD, ainsi qu'un gène désigné CHV ORF2 ont été décrits (K. Limbach et al. J. Gen. Virol. 1994. 75. 2029-2039).

A la suite de leurs travaux sur CHV, les inventeurs ont réussi à déterminer plusieurs régions non essentielles pour la réplication *in vitro,* lesquelle se sont révélées utiles pour la construction de virus CHV recombinés. Ces régions non essentielles pour la réplication sont le cadre ouvert de lecture 3 et le cadre de lecture 5 de CHV. EP 0 447 303 avait déjà décrit la construction d'un vecteur viral à partir d'un herpèsvirus (y compris le CHV) par insertion d'un acide nucléique hétérologue dans le gène codant pour la petite sous-unité RR2 de la ribonucléotide réductase, qui a été déterminé comme un site non-essentiel pour la réplication. Les inventeurs sont donc en mesure de proposer le virus CHV en tant que vecteur de vaccination pour le chien. On a trouvé que les vecteurs vaccinaux selon l'invention présentaient des avantages particuliers pour la vaccination du chien. En effet, l'herpèsvirus canin est très spécifique d'espèce et possède un génome de grande taille contenant plusieurs sites potentiels d'insertion et permettant l'insertion simultanée de plusieurs cassettes d'expression pour des gènes étrangers. Cela offre la possibilité de vacciner les chiens en même temps contre différents agents pathogènes canins en utilisant un seul virus recombiné.

L'objectif principal de l'invention est de fournir un vecteur vaccinal permettant l'expression d'immunogènes de pathogènes canins en vue de la protection des chiens contre les principales maladies infectieuses canines.

Un autre objectif de l'invention est de fournir un tel vecteur permettant la vaccination des chiens, et en particulier des chiots ayant des anticorps maternels, par voie mucosale, notamment orale, nasale ou conjonctivale.

Un autre objectif encore de l'invention est de fournir un tel vecteur qui permette en même temps la vaccination contre l'herpèsvirose chez le chiot.

La présente invention a donc pour objet un vaccin vivant recombinant utilisant comme vecteur un herpèsvirus canin comprenant, et exprimant, au moins une séquence nucléotidique codant pour un polypeptide, cette séquence étant insérée dans un site non essentiel pour la réplication *in vitro.*

Les inventeurs ont isolé et analysé un fragment génomique du virus CHV sur lequel ils ont caractérisé 5 cadres ouverts de lecture (COL1 à COL5) parmi lesquels deux (COL3 et COL5) se sont révélés non essentiels pour la réplication *in vitro.* Par ailleurs, d'autres régions du génome CHV peuvent également être utilisées pour insérer des gènes étrangers. Ces sites d'insertion sont : gène thymidine kinase (COL CHV TK) (Rémond M. et al. Virus Research. 1995. 39. 341-354) et séquence située entre le COL CHV orf19 (ou COL19) et le COL CHV orf22 (ou COL22)(Rémond M. et al. J. Gen. Virol. 1996. 76. 37-48). Ces sites sont décrits plus précisément dans les exemples de la présente invention.

De préférence, la séquence insérée code pour un polypeptide antigénique et, préférentiellement, pour un polypeptide antigénique d'un agent pathogène canin. On peut également insérer des séquences codant pour des protéines immunomodulatrices telles que des cytokines. Selon une modalité avantageuse, on peut associer une séquence codant pour une cytokine, ou analogue, et une séquence codant pour un antigène. Si besoin est, plusieurs séquences de cytokines peuvent être associées entre elles, éventuellement en combinaison avec une ou des séquences codant pour des antigènes.

L'insertion dans les sites est réalisée par insertion simple (sans délétion) ou après délétion partielle ou totale du ou des COLs utilisés comme sites d'insertion.

Comme virus parental pour la construction de virus CHV recombinés, on peut utiliser en particulier la souche CHV F205 qui a été isolée par L. Carmichael (Proc. Soc. Exp. Biol. Med. 1965. 120. 644-650).

Pour l'expression des gènes étrangers insérés sur le génome de CHV selon la présente invention, on utilisera de préférence un promoteur eucaryote fort, tel que, préférentiellement, un promoteur cytomegalovirus (CMV) immediate early (IE). Par promoteur CMV IE, on entend notamment un fragment tel que donné dans les exemples ainsi que ses sous-fragments conservant la même activité promotrice. Le promoteur CMV IE peut être le promoteur humain (HCMV IE) ou le promoteur murin (MCMV IE), ou encore un promoteur CMV IE d'une autre origine, par exemple CMV du rat, du cobaye, ou du porc.

On pourra insérer au moins deux séquences nucléotidiques dans un site, sous le contrôle de promoteurs différents. Il pourra notamment s'agir de promoteurs CMV IE d'origines différentes.

Selon un développement avantageux de l'invention, on associe au promoteur CMV IE un autre promoteur de telle façon que les deux promoteurs aient leurs extrémités 5' adjacentes et que les transcriptions initiées à partir de ces deux promoteurs se fassent en sens opposés. Cette disposition particulière permet d'insérer, dans un même site, deux séquences nucléotidiques, l'une sous la dépendance du promoteur CMV IE, l'autre sous celle du promoteur associé. Cette construction est remarquable par le fait que la présence du promoteur CMV IE, et notamment de sa partie activatrice (en anglais "enhancer"), peut activer la transcription induite par le promoteur associé. Comme promoteur associé, on peut citer, par exemple, un promoteur CMV d'espèce différente du premier promoteur. On peut également envisager d'autres promoteurs tels que le promoteur RNA1.8 du virus de la maladie de Marek (MDV) (G. Bradley et al. J. Virol. 1989. 63.2534-2542).

La séquence nucléotidique insérée dans le vecteur CHV pour être exprimée peut être toute séquence codant pour un polypeptide antigénique d'un agent pathogène canin capable, une fois exprimée dans les conditions favorables procurées par l'invention, d'assurer une immunisation conduisant à une protection efficace de l'animal vacciné contre l'agent pathogène. On pourra donc insérer, dans les conditions décrites par la présente l'invention, les séquences nucléotidiques codant pour les antigènes d'intérêt pour une maladie donnée, notamment les maladies virales, bactériennes ou parasitaires citées plus haut.

Le cas typique de l'invention est l'insertion d'au moins une séquence nucléotidique codant convenablement pour un polypeptide du virus de la maladie de Carré (Canine Distemper Virus = CDV) et, de préférence, pour le polypeptide CDV HA (Sidhu M. et al., Virology. 1993. 193. 66-72) ou pour le polypeptide CDV F (Barrett T. et al. Virus Research. 1987. 8. 373-386). On peut également insérer ces deux gènes ensemble dans le vecteur CHV. On obtient ainsi un vaccin vivant recombinant assurant une protection contre la maladie de Carré.

D'autres cas préférés de l'invention sont l'insertion de séquences nucléotidiques codant pour des antigènes ou des fragments d'antigènes du virus de la rage, en particulier le gène G (Brevets FR-A-2 515 685 et EP-A-162 757), du virus de la parvovirose canine (gène VP2)(Parrish C. et al. J. Virol. 1991. 65. 6544-6552), du virus parainfluenza de type 2 (gènes HA et/ou F). On peut également insérer des séquences codant pour des antigènes de *Borrelia burgdorferi,* en particulier les gènes codant pour les antigènes OspA et OspB (Bergström S. et al. Mol. Microbiol. 1989. 3. 479-486).

Un cas typique de l'invention est un vaccin comprenant une séquence nucléotidique codant pour un antigène du virus de la maladie de Carré sous le contrôle de CMV IE et une séquence nucléotidique codant pour un antigène d'une autre maladie virale canine, notamment celles citées plus haut, sous le contrôle de l'autre promoteur.

Bien entendu, les séquences hétérologues et leurs promoteurs associés peuvent être insérés plus classiquement en tandem dans le locus d'insertion, c'est-à-dire suivant un même sens de transcription.

L'expression de plusieurs gènes hétérologues insérés dans le locus d'insertion peut être également possible par insertion d'une séquence appelée "IRES" (Internal Ribosome Entry Site) provenant notamment d'un picornavirus tel que le virus de la maladie vésiculaire du porc (swine vesicular disease virus, SVDV; B.-F. Chen et al., J. Virology, 1993, 67, 2142-2148), le virus de l'encéphalomyocardite (EMCV; R.J. Kaufman et al., Nucleic Acids Research, 1991, 19, 4485-4490), le virus de la fièvre aphteuse (FMDV; N. Luz et E. Beck, J. Virology, 1991, 65, 6486-6494), ou encore d'une autre origine. Le contenu de ces trois articles est incorporé par référence. La cassette d'expression de deux gènes aurait donc la structure minimale suivante: promoteur - gène 1 - IRES - gène 2 - signal de polyadénylation. Le vaccin vivant recombinant selon l'invention pourra donc comprendre, insérée dans le locus d'insertion, une cassette d'expression comprenant successivement un promoteur, deux ou plusieurs gènes séparés deux à deux par un IRES, et un signal de polyadénylation.

En plus de l'insertion dans le locus selon l'invention, on peut réaliser une ou plusieurs autres insertions, une ou plusieurs mutations, ou une ou plusieurs délétions ailleurs dans le génome. Dans tous les cas, l'insertion dans un autre locus que celui décrit dans l'invention, permet d'exprimer d'autres gènes.

L'utilisation des virus recombinants selon l'invention permet de protéger les chiens contre une ou plusieurs des maladies citées plus haut et, en même temps, contre l'herpèsvirose canine.

La présente invention a aussi pour objet une formule de vaccin multivalent, comprenant, en mélange ou à mélanger, au moins deux vaccins vivants recombinants tels que définis plus haut, ces vaccins comprenant des séquences insérées différentes, isolées notamment de pathogènes différents. Ces formules de vaccins contiennent des dosages et/ou des véhicules adaptés à la voie d'administration.

La présente invention a aussi pour objet les virus CHV modifiés dans l'un au moins des sites indiqués.

Elle a aussi pour objet une méthode de vaccination, en particulier des chiens, dans laquelle on administre par toute voie parentérale ou mucosale, mais de préférence par voie mucosale, notamment orale et/ou nasale, une quantité efficace d'un vaccin tel que défini plus haut. La dose vaccinale sera comprise, de préférence, entre 10² DICC50 et 10⁷ DICC50. De préférence, la dose pour la voie parentérale sera comprise entre 10⁴ DICC50 et 10⁷ DICC50, et pour la voie orale et/ou nasale, entre 10² DICC50 et 10⁵ DICC50. Tel que défini, le vaccin est efficace en général après une seule administration par voie orale et/ou nasale. Cependant, des administrations répétées peuvent être nécessaires.

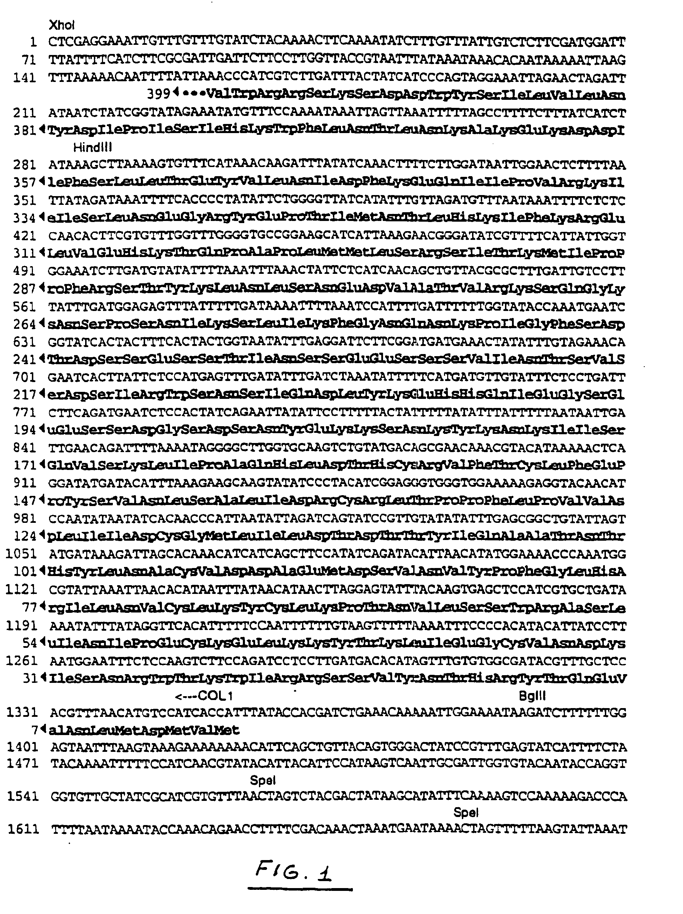

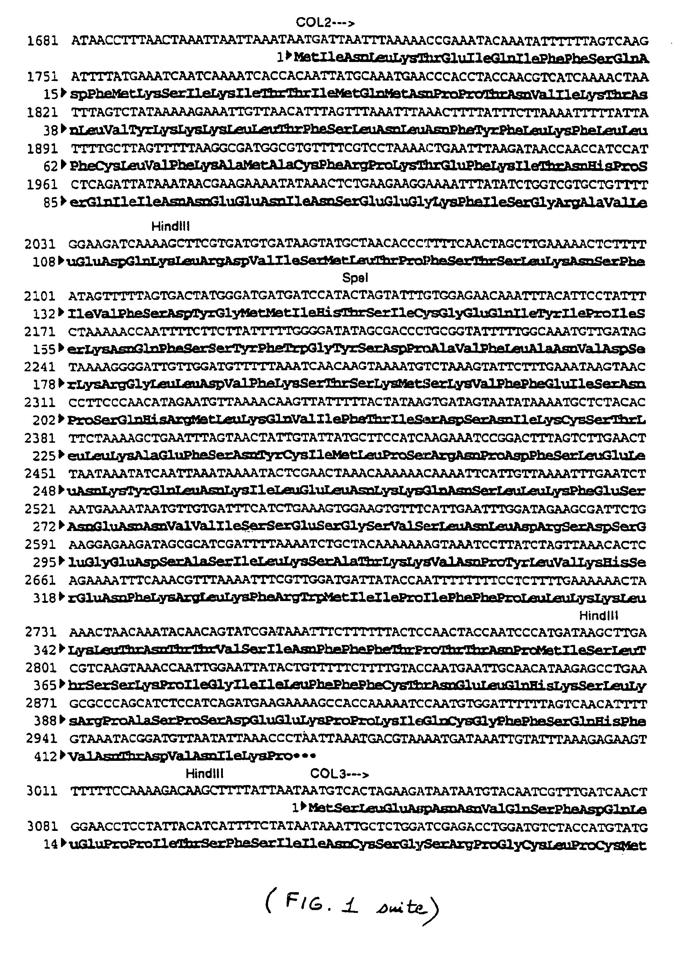

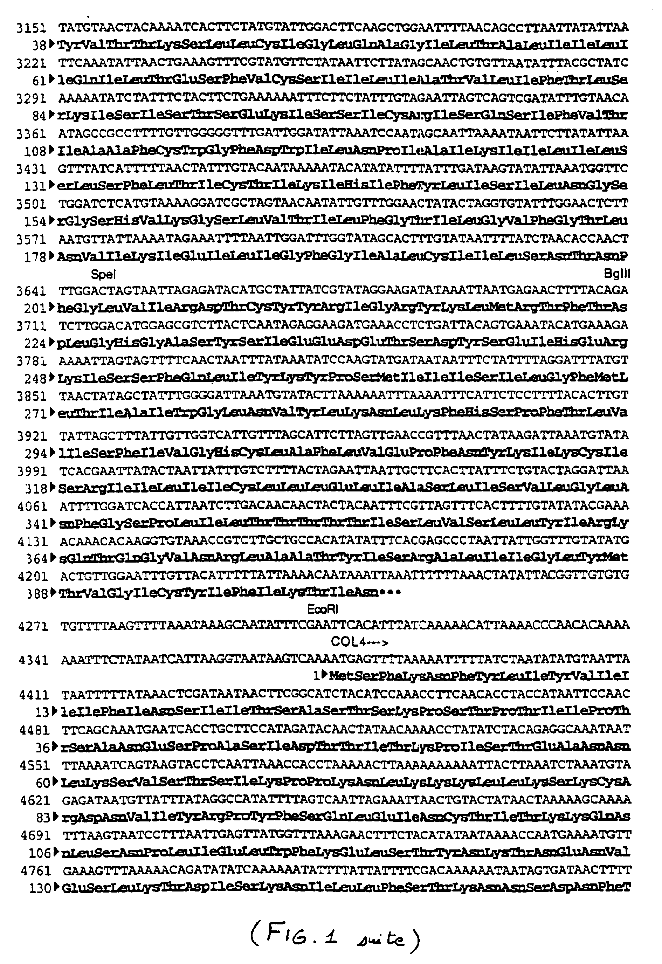

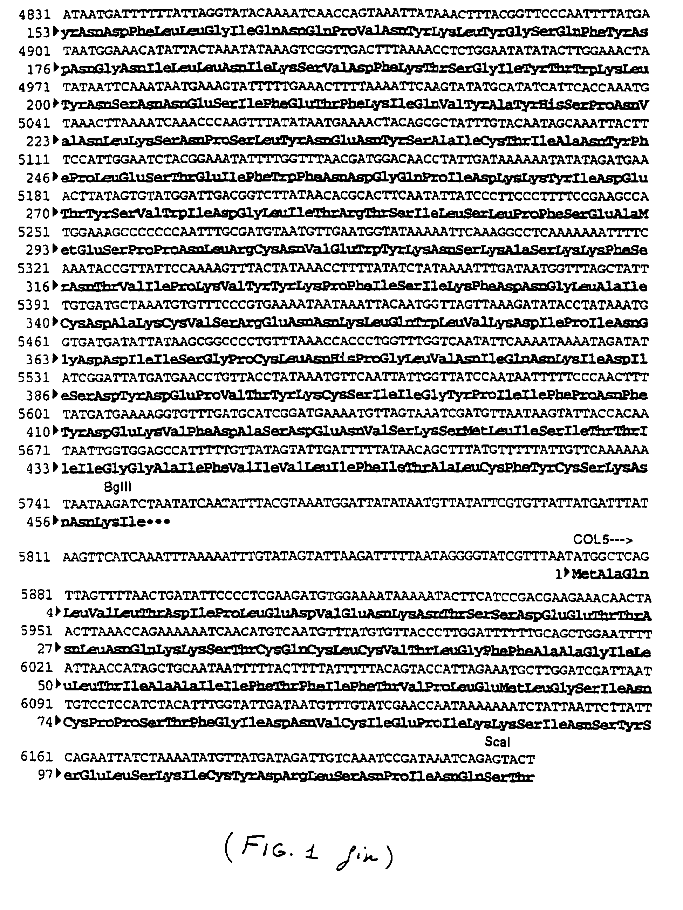

La présente invention decrit également des fragments d'ADN comprenant tout ou partie de la séquence définie par les positions 1 à 6216 sur SEQ ID N°1 (Figure N°1), notamment tout ou partie du site COL3 défini et/ou des séquences flanquantes localisées en amont et en aval de ce site, fragments qui seront utiles comme bras flanquants pour les techniques de recombinaison homologue avec le génome du virus CHV choisi comme virus parental. Bien entendu, l'invention divulgue aussi les variantes de ces fragments qui correspondent aux séquences équivalentes des autres souches de CHV. Le spécialiste a toute latitude pour choisir les régions servant de bras flanquants en liaison avec le type d'insertion (avec ou sans délétion) ou de délétion (partielle ou totale) choisi. D'une manière générale, les bras flanquants pourront ainsi avoir de 100 à 800 paires de bases, mais peuvent être de plus grande taille si nécessaire.

L'invention décrit aussi une méthode de préparation des vecteurs et vaccins selon l'invention telle qu'elle ressort de la description des vaccins, par insertion de gènes d'intérêts dans le site d'insertion.

L'invention va être maintenant décrite plus en détail à l'aide d'exemples de réalisation non limitatifs, pris en référence au dessin, dans lequel :
- **Figure 1 :**: Séquence de la région CHV (6216 paires de bases) et traduction des différents cadres ouverts de lecture (ORF) présents sur cette séquence (COL1 à COL5).
- **Figure 2 :**: Plasmide pPB200 (plasmide donneur pour l'insertion de cassettes d'expression dans le site CHV COL3).
- **Figure 3 :**: Construction du plasmide pPB202 (plasmide donneur pour l'insertion de cassettes d'expression dans le site CHV COL5).
- **Figure 4 :**: Construction du plasmide pPB204 (plasmide donneur pour l'insertion de cassettes d'expression dans le site CHV TK).
- **Figure 5 :**: Construction du plasmide pPB206 (plasmide donneur pour l'insertion de cassettes d'expression dans le site situé entre les gènes CHV orf19 et CHV orf 22), (pas l'objet de l'invention)
- **Figure 6 :**: Construction du plasmide pPB208 (cassette d'expression pour le gène CDV HA).
- **Figure 7 :**: Construction du plasmide pPB210 (cassette d'expression pour le gène CDV F).
- **Figure 8 :**: Construction du plasmide pPB213 (plasmide donneur pour l'insertion de la cassette d'expression du gène CDV HA dans le site CHV COL3).
- **Figure 9 :**: Construction du plasmide pPB214 (plasmide donneur pour l'insertion de la cassette d'expression du gène CDV F dans le site CHV COL3).
- **Figure 10 :**: Plasmide PB200'
- **Figure 11 :**: Construction du plasmide pPB212 (cassette d'expression du gène G du virus de la rage).
- **Figure 12 :**: Construction du plasmide pPB215 (plasmide donneur pour l'insertion de la cassette d'expression du gène G du virus de la rage dans le site CHV COL3).
- **Figure 13 :**: Construction du plasmide pPB216 (plasmide donneur pour l'insertion de la cassette d'expression du gène CDV HA dans le site CHV COL5).
- **Figure 14 :**: Construction du plasmide pPB217 (plasmide donneur pour l'insertion de la cassette d'expression du gène CDV HA dans le site CHV TK). (Pas l'objet de l'invention)
- **Figure 15 :**: Construction du plasmide pPB218 (plasmide donneur pour l'insertion de la cassette d'expression du gène CDV HA le site situé entre les gènes CHV orf19 et CHV orf22, (pas l'objet de l'invention)

### 2. EXEMPLES

Toutes les constructions de plasmides ont été réalisées en utilisant les techniques standards de biologie moléculaire décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention ont été isolés en utilisant le kit "Geneclean" (BI0101 Inc. La Jolla, CA).
Le virus utilisé comme virus parental est la souche F205 (appelée aussi souche Carmichael) de l'herpèsvirus canin. Cette souche a été obtenue du Dr. L. Carmichael (Cornell University, NY) qui l'a isolée et a décrit ses caractéristiques biologiques (Proc. Soc. Exp. Biol. Med. 1965. 120. 644-650). Les conditions de culture de ce virus sont les suivantes : des cellules MDCK (Madin Darbey Canine Kidney ATCC CCL34), cultivées en milieu minimum essentiel de Eagle (milieu "MEM) sont inoculées avec la souche CHV F205 en utilisant une multiplicité d'infection de 1. Les cellules infectées sont alors incubées à 37°C pendant environ 36 heures, jusqu'à apparition d'un effet cytopathique complet.

### Exemple 1: Extraction de l'ADN de l'herpèsvirus canin:

Après culture, le surnageant et les cellules lysées sont récoltés et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EUTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN précipité est centrifugé à 10000 g pendant 15 minutes à + 4°C. Le culot d'ADN est séché, puis repris dans un volume minimum d'eau ultrapure stérile.

### Exemple 2: Clonage et caractérisation de la région CHV COL1 - COL5

L'ADN génomique purifié de la souche F205 du virus CHV a été digéré avec les enzymes de restriction Scal et *Xho*I et le fragment Scal-Xhol d'environ 6200 pb a été cloné dans le vecteur pBlueScript SKII + (Stratagene Ref. 212205), préalablement digéré par Scal et *Xho*I*,* pour donner le plasmide pPB154.
Le fragment XhoI-ScaI cloné dans le plasmide pPB154 a été entièrement séquencé sur les deux brins pour générer la séquence de 6216 pb de la figure 1.

Plusieurs cadres ouverts de lecture de taille supérieure à 65 acides aminés ont été identifiés sur cette séquence (Figure 1) :
Le premier cadre de lecture (COL1) (positions 1353 - 157) se trouve sur le brin complémentaire et code pour un polypeptide de 398 acides aminés.
Le deuxième cadre de lecture (COL2) (positions 1708 - 2970) code pour un polypeptide de 420 acides aminés.
Le troisième cadre de lecture (COL3) (positions 3040 - 4242) code pour un polypeptide de 400 acides aminés.
Le quatrième cadre de lecture (COL4) (positions 4374 - 5753) code pour un polypeptide de 459 acides aminés.
Le cinquième cadre de lecture (COL5) (positions 5872 - 6216) est incomplet et code pour un protéine tronquée de 115 acides aminés.

Les différents cadres ouverts de lecture sont rassemblés dans le tableau ci-dessous :

| Cadre ouvert de lecture | Début - Fin (positions sur figure 1) | Taille en acides aminés |
|---|---|---|
| COL 1 | 1353 - 157 | 398 aa |
| COL 2 | 1708 - 2970 | 420 aa |
| COL 3 | 3040 - 4242 | 400 aa |
| COL 4 | 4374 - 5753 | 459 aa |
| COL 5 | 5872 - 6216 | 115 aa |

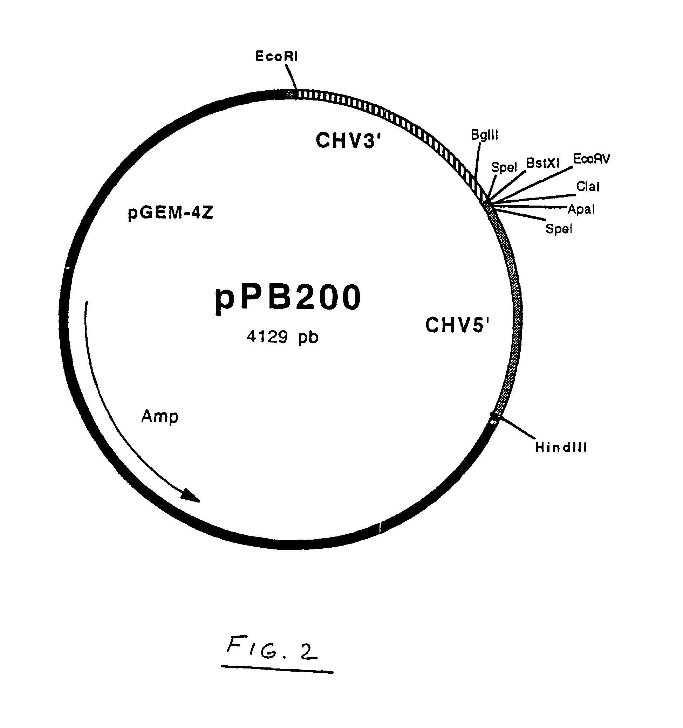

### Exemple 3 : Construction du plasmide pPB200 pour l'insertion de cassettes d'expression dans le site CHV COL3 (Figure 2)

Le plasmide pPB154 (9121 pb) (exemple 2) a été digéré par *Hin*dIII et *Spel* pour isoler le fragment HindIII-SpeI de 620 pb (fragment A). Le plasmide pPB154 a été digéré par *EcoRI* et *Spel* pour isoler le fragment EcoRI-Spel de 659 pb (fragment B). Les fragments A et B ont été ligaturés ensemble avec le vecteur pGEM4Z (Promega Ref. P2161), préalablement digéré par *EcoRI* et *Hind*lll, pour donner le plasmide pPB199 (4096 pb). Le plasmide pPB199 a ensuite été digéré par *Spe*l, traité par la phosphatase alcaline, puis ligaturé avec le site multiple de clonage, obtenu par hybridation des 2 oligonucléotides suivants:
JCA070 (33 mer).
5'CTAGTCCAGCAAGGTGGATCGATATCGGGCCCA 3'
JCA071 (33 mer).
5'CTAGTGGGCCCGATATCGATCCACCTTGCTGGA 3'
pour donner le plasmide pPB200 (4129 pb).

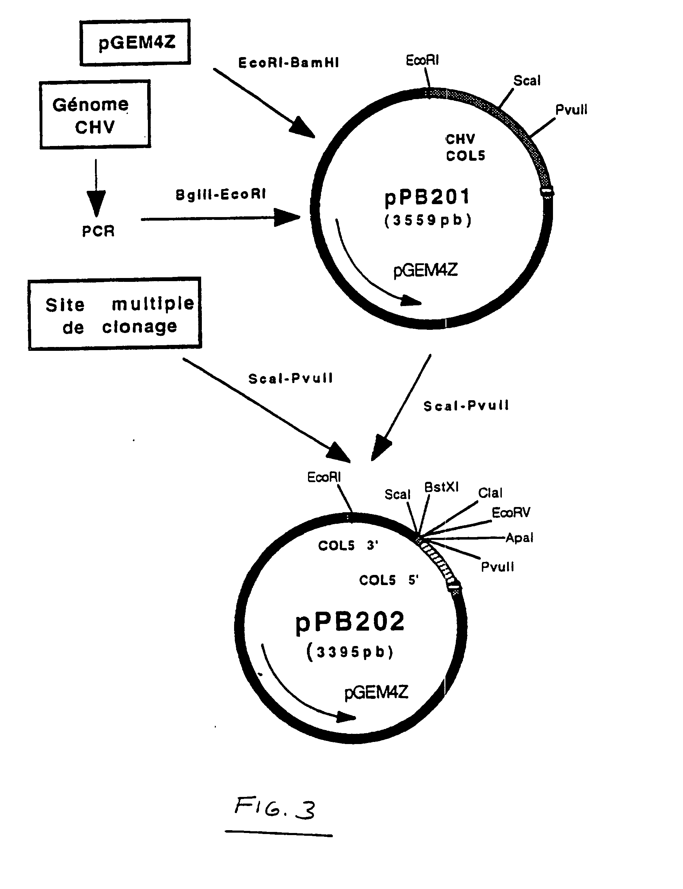

### Exemple 4: Construction du plasmide pPB202 pour l'insertion de cassettes d'expression dans le site CHV COL5 (Figure 3)

La séquence du gène CHV COL5 a été publiée récemment (Limbach K. et al. J. Gen. Virol. 1994. 75. 2029-2039). Une réaction PCR a été réalisée avec l'ADN génomique du virus CHV souche F205 (exemple 1) et avec les oligonucléotides suivants:
JCA072 (22 mer)
5'CAGCTTTATGTTTTTATTGTTC 3'
JCA073 (29 mer)
5'AAAGAATTCTACAACTGTTTAATAAAGAC 3'
pour obtenir un fragment PCR de 751 pb contenant le gène CHV ORF2 complet. Ce fragment a été digéré par *Bgl*II et *EcoR*I pour isoler un fragment BgIII-EcoRI de 709 pb. Ce fragment a été ligaturé avec le vecteur pGEM4Z (Promega Ref. P2161), préalablement digéré par *EcoRI* et *BamH*I, pour donner le plasmide pPB201 (3559 pb). Le plasmide pPB201 a ensuite été digéré par *Sca*I et *Pvu*II, puis ligaturé avec un site multiple de clonage obtenu par hybridation des 2 oligonucléotides suivants:
JCA074 (36 mer)
5'ACTCCAGCTACATGGGATATCGGGCCCATCGATCAG 3'
JCA075 (36 mer)
5'CTGATCGATGGGCCCGATATCCCATGTAGCTGGAGT 3'
pour donner le plasmide pPB202 (3395 pb).

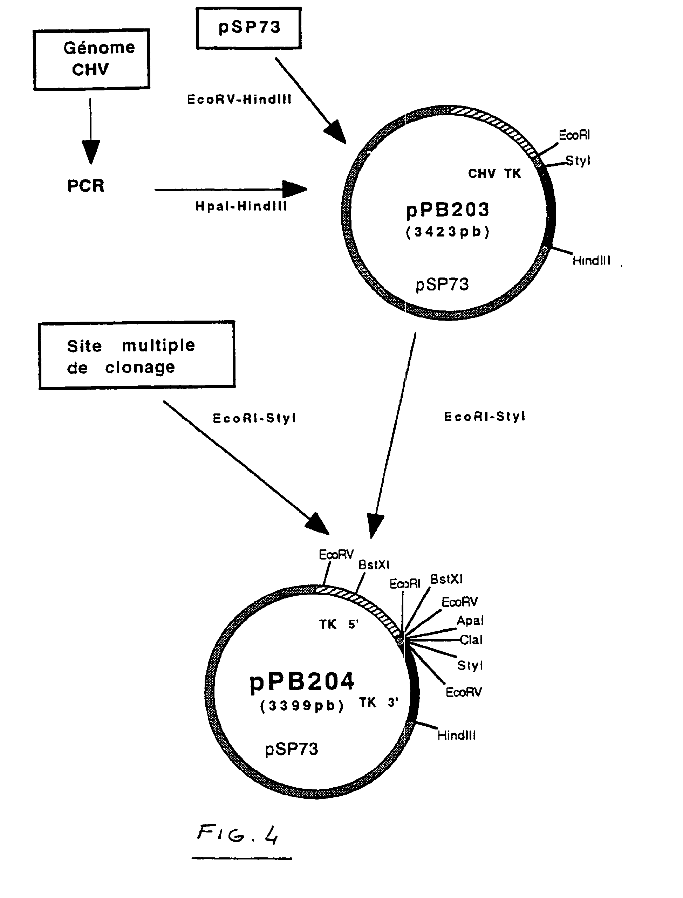

### Exemple 5: Construction du plasmide pPB204 pour l'insertion de cassettes d'expression dans le site CHV TK (Figure 4) (pas l'objet de l'invention)

La séquence du gène CHV thymidine kinase (TK) a été publiée récemment (M. Rémond M. et al. Virus Research. 1995. 39. 341-3541. Une réaction PCR a été réalisée avec l'ADN génomique du virus CHV souche F205 (exemple 1) et avec les oligonucléotides suivants:
JCA076 (35 mer)
5'AGCGTTAACCTCAAAAGCCAAATTTACACTTCCCG 3'
JCA077 (38 mer)
5'CCCAAGCTTTTCTAAAGCCCATTTATAAATAATAAATG 3'
pour obtenir un fragment PCR de 1030 pb contenant le gène thymidine kinase (TK). Ce fragment a été digéré par *Hpa*I et *Hind*III pour isoler un fragment Hpal-Hindlll de 1019 pb. Ce fragment a été ligaturé avec le vecteur pSP73 (Promega Ref. P2221), préalablement digéré par *EcoRV* et *Hin*dIII, pour donner le plasmide pPB203 (3423 pb).
Le plasmide pPB203 a ensuite été digéré par *EcoR*I et *Sty*I, puis ligaturé avec un site multiple de clonage, obtenu par hybridation des 2 oligonucléotides suivants: JCA078 (36 mer)
5'AATTCCCAGCTACATGGGATATCGGGCCCATCGATC 3'
JCA079 (36 mer)
5'CAAGGATCGATGGGCCCGATATCCCATGTAGCTGGG 3'
pour donner le plasmide pPB204 (3399 pb).

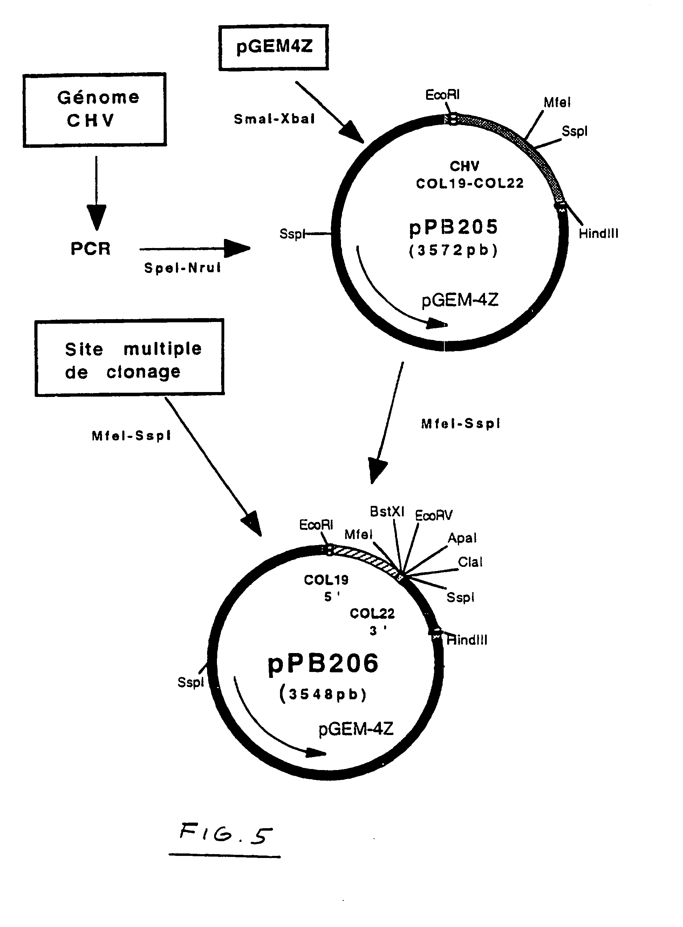

### Exemple 6: Construction du plasmide pPB206 pour l'insertion de cassettes d'expression dans le site situé entre les gènes CHV orf19 et orf22 (Figure 5)

### (pas l'objet de l'invention)

La séquence de la région intergénique correspondant à la délétion naturelle des gènes codant pour la grande sous-unité (gène "RR1") et pour la petite sous-unité (gène "RR2") de la ribonucléotide réductase a été publiée récemment (Rémond M. et al. J. Gen. Virol. 1996. 77. 37-48). Selon la nomenclature utilisée par Rémond *et al*., la délétion de ces deux gènes se trouve entre les cadres ouverts de lecture dénommés CHV "orf19" et CHV "orf22"). Une réaction PCR a été réalisée avec l'ADN génomique du virus CHV souche F205 (exemple 1) et avec les oligonucléotides suivants:
JCA080 (36 mer)
5'GGAGATCTAGTAAATTAAATAGTAATTCATTTAATG 3'
JCA081 (33 mer)
5'CAGTCGCGAAGATGAAAATAAAATCCATCGAAG 3'
pour obtenir un fragment PCR de 720 pb contenant la région intergénique correspondant à la délétion naturelle des gènes CHV "orf 19" et "orf22". Ce fragment a été digéré par *Spe*I et *Nru*I pour isoler un fragment SpeI-NruI de 709 pb. Ce fragment a été ligaturé avec le vecteur pGEM4Z (Promega Ref. P2161), préalablement digéré par *Sma*I et *Xba*I*,* pour donner le plasmide pPB205 (3572 pb). Le plasmide pPB205 a été ensuite digéré par *Mfe*I, puis partiellement digéré par *Ssp*I, afin d'isoler le fragment Mfel-Sspl de 3512 pb. Ce fragment a alors été ligaturé avec un site multiple de clonage, obtenu par hybridation des 2 oligonucléotides suivants:
JCA082 (38 mer)
5'AATTGGCAGCTACATGGGATATCGGGCCCATCGATAAT 3'
JCA083 (34 mer)
5'ATTATCGATGGGCCCGATATCGGATGTAGCTGGC 3'
pour donner le plasmide pPB206 (3548 pb).

### Exemple 7: Isolement de l'ARN génomique de la souche CDV Onderstepoort et clonage de l'ADN complémentaire codant pour les gènes HA et F

La souche CDV Onderstepoort (Mitchell W. et al. J. Virol. Meth. 1987. 18. 121-131) a été cultivée sur cellules MDCK (Madin Darbey Canine Kidney) en milieu DMEM (Gibco). Après purification du virus, l'ARN viral génomique a été isolé en utilisant la technique d'extraction au thiocyanate de guanidium/phénol-chloroforme (Chomczynski P. et Sacchi N., Anal. Biochem. 1987. 162. 156-159). Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (respectivement gènes HA et F). La réaction de transcription inverse (RT) et l'amplification en chaîne par polymérase (PCR) ont été effectuées selon les techniques standards (Sambrook J. *et al.* 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction et cloné dans le vecteur approprié.

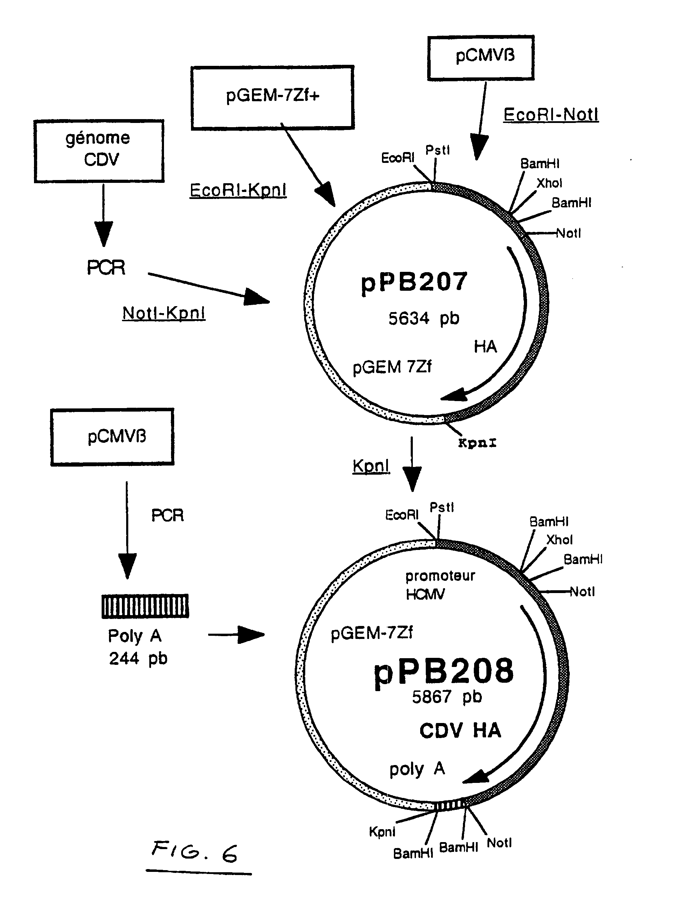

### 7.1. Construction de la cassette d'expression CDV HA (pPB208) (Figure 6)

Le plasmide pCMVB (Clontech Ref. 6177-1) a été digéré par *EcoR*I et *Not*I pour isoler un fragment EcoRI-Notl de 818 pb contenant la région promotrice du gène immediate-early du cytomégalovirus humain (fragment A).
Une réaction RT-PCR a été réalisée avec l'ARN génomique du virus CDV (souche Onderstepoort) et avec les oligonucléotides suivants:
JCA084 (32 mer)
5'TTGCGGCCGCATGCTCCCCTACCAAGACAAGG 3'
JCA085 (28 mer)
5'TTGGTACCTTAACGGTTACATGAGAATC 3'
pour obtenir un fragment PCR de 1837 pb contenant le gène HA du CDV. Ce fragment a été digéré par *Not*I et *Kpn*I pour isoler un fragment Notl-Kpnl de 1826 pb (fragment B).
Les fragments A et B ont été ligaturés ensemble avec le vecteur pGEM-7Zf + (Promega Cat # P2251), préalablement digéré par *EcoRl* et *Kpnl,* pour donner le plasmide pPB207 (5634 pb).
Une réaction PCR a été réalisée avec le plasmide pCMVB et avec les oligonucléotides suivants:
PB088 (30 mer)
5'TTGGGTACCGCCTCGACTCTAGGCGGCCGC 3'
PB089 (32 mer)
5'TTGGGTACCGGATCCGAAAAAACCTCCCACAC 3'
pour obtenir un fragment PCR de 244 pb contenant le signal de polyadénylation du gène précoce du virus SV40. Ce fragment a été digéré par *Kpn*I pour isoler un fragment Kpnl-Kpnl de 233 pb. Ce fragment a ensuite été ligaturé avec le plasmide pPB207, préalablement digéré par *Kpn*I, pour donner le plasmide pPB208 (5867 pb).

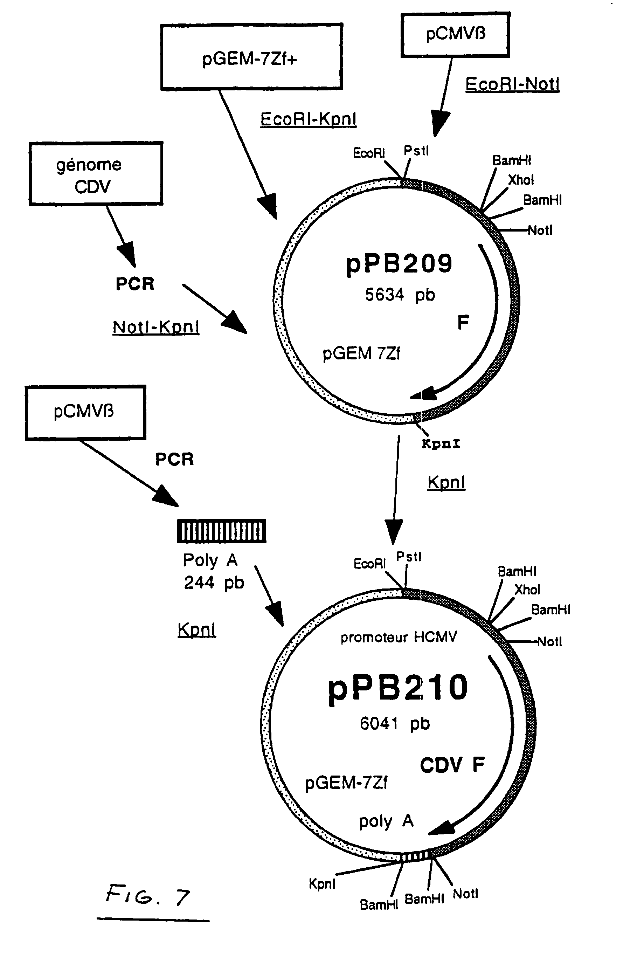

### 7.2. Construction de la cassette d'expression CDV F (pPB210) (Figure 7)

Le plasmide pCMVB (Clontech Ref. 6177-1) a été digéré par *EcoR*I et *Not*I pour isoler un fragment EcoRI-Notl de 818 pb contenant la région promotrice du gène immediate-early du cytomégalovirus humain (fragment A).
Une réaction RT-PCR a été réalisée avec l'ARN génomique du virus CDV (souche Onderstepoort) et avec les oligonucléotides suivants:
JCA086 (34 mer)
5'TTGCGGCCGCATGCACAGGGGAATCCCCAAAAGC 3'
JCA087 (28 mer)
5'TTGGTACCTCAGAGTGATCTCACATAGG 3'
pour obtenir un fragment PCR de 2011 pb contenant le gène F du CDV. Ce fragment a été digéré par *Not*I et *Kpn*I pour isoler un fragment Notl-Kpnl de 2000 pb (fragment B). Les fragments A et B ont été ligaturés ensemble avec le vecteur pGEM-7Zf + (Promega Ref. P2251), préalablement digéré par *EcoRI* et *Kpn*I, pour donner le plasmide pPB209 (5808 pb).

Une réaction PCR a été réalisée avec le plasmide pCMVB et avec les oligonucléotides suivants:
PB088 (30 mer)
5'TTGGGTACCGCCTCGACTCTAGGCGGCCGC 3'
PB089 (32 mer)
5'TTGGGTACCGGATCCGAAAAAACCTCCCACAC 3'
pour obtenir un fragment PCR de 244 pb contenant le signal de polyadénylation du gène précoce du virus SV40. Ce fragment a été digéré par *Kpn*I pour isoler un fragment Kpnl-Kpnl de 233 pb. Ce fragment a ensuite été ligaturé avec le plasmide pPB209 préalablement digéré par KpnI pour donner le plasmide pPB210 (6041 pb).

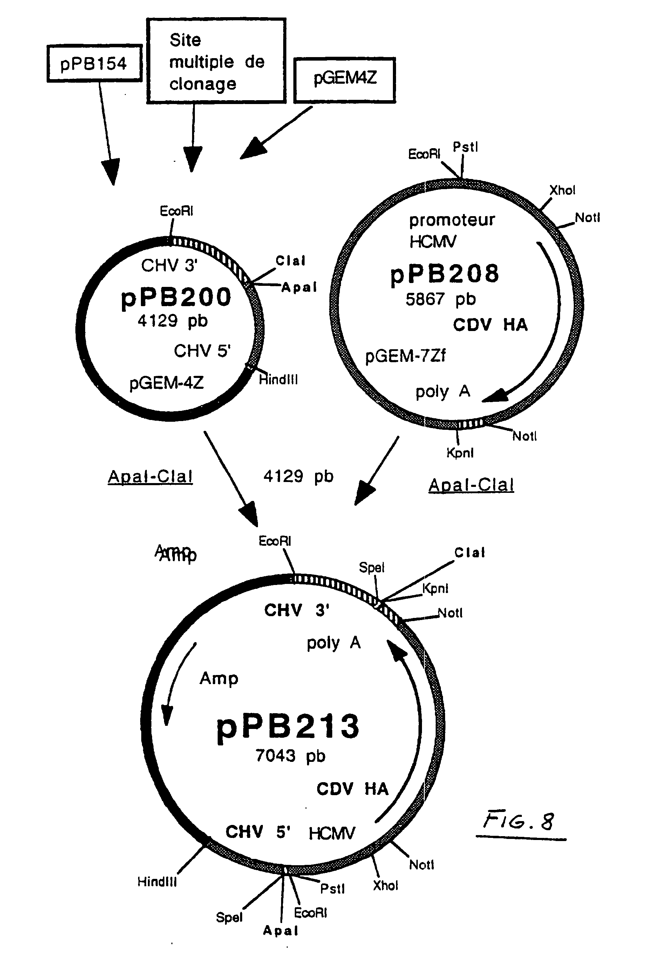

### Exemple 8: Construction du plasmide donneur pPB213 pour l'insertion de la cassette d'expression CDV HA dans le site CHV COL3 (Figure 8)

Le plasmide pPB208 (exemple 7.1.) a été digéré par *Apa*I et *Cla*I pour isoler un fragment Apal-Clal de 2920 pb contenant la cassette d'expression du gène HA du virus CDV. Ce fragment a été ensuite ligaturé avec le plasmide pPB200 (exemple 3), préalablement digéré par *Apa*I et *Cla*I, pour donner le plasmide pPB213 (7043 pb). Ce plasmide permet l'insertion de la cassette d'expression du gène CDV HA dans le site CHV COL3.

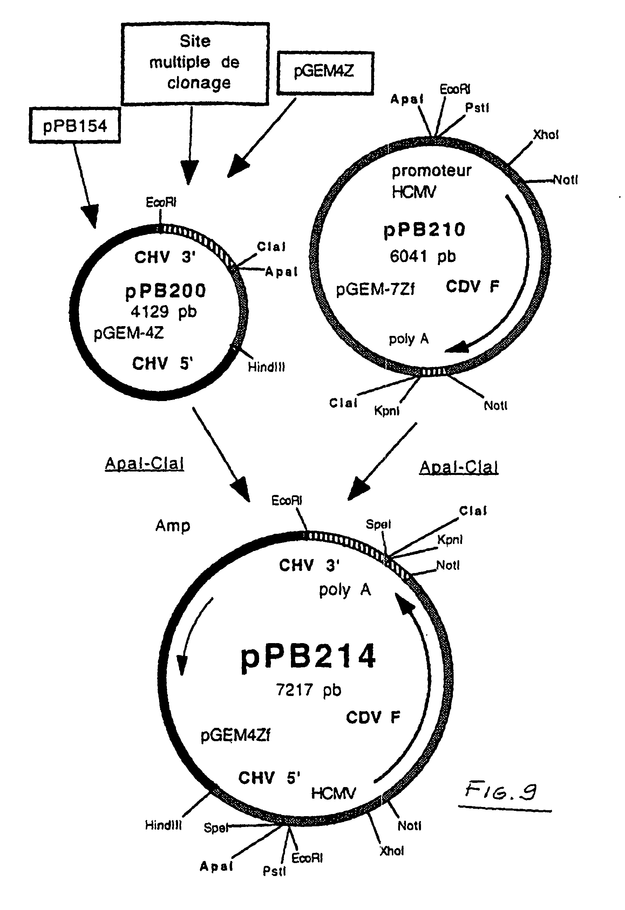

### Exemple 9: Construction du plasmide donneur pPB214 pour l'insertion de la cassette d'expression CDV F dans le site CHV COL3 (Figure 9)

Le plasmide pPB210 (exemple 7.2.) a été digéré par *Apa*I et *Cla*I pour isoler un fragment ApaI-ClaI de 3100 pb contenant la cassette d'expression du gène F du virus CDV. Ce fragment a été ensuite ligaturé avec le plasmide pPB200 (exemple 3), préalablement digéré par *Apa*I et *Cla*I, pour donner le plasmide pPB214 (7217 pt . Ce plasmide permet l'insertion de la cassette d'expression du gène CDV F dans le site CHV COL3.

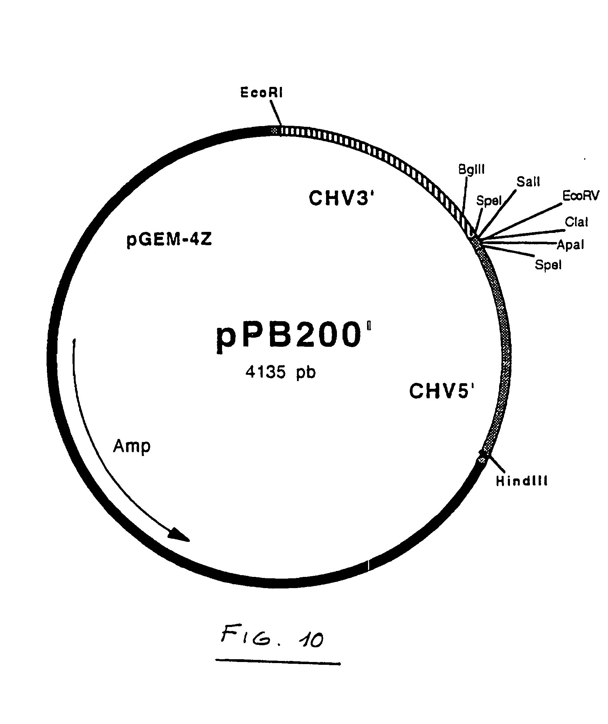

### Exemple 10: Construction du plasmide donneur PB215 pour l'insertion de la cassette d'expression du gène G du virus de la rage dans le site CHV COL3 (Figures 10, 11 et 12)

Le plasmide pPB199 (exemple 3) a été digéré par *Spe*I, traité par la phosphatase alcaline, puis ligaturé avec le site multiple de clonage, obtenu par hybridation des 2 oligonucléotides suivants:
JCA088 (39 mer)
5'CTAGTCCAGCAAGGTGTCGACGGATCGATATCGGGCCCA 3'
JCA089 (39 mer)
5'CTAGTGGGCCCGATATCGATCCGTCGACACCTTGCTGGA 3'
pour donner le plasmide pPB200' (4135 pb) (Figure 10).

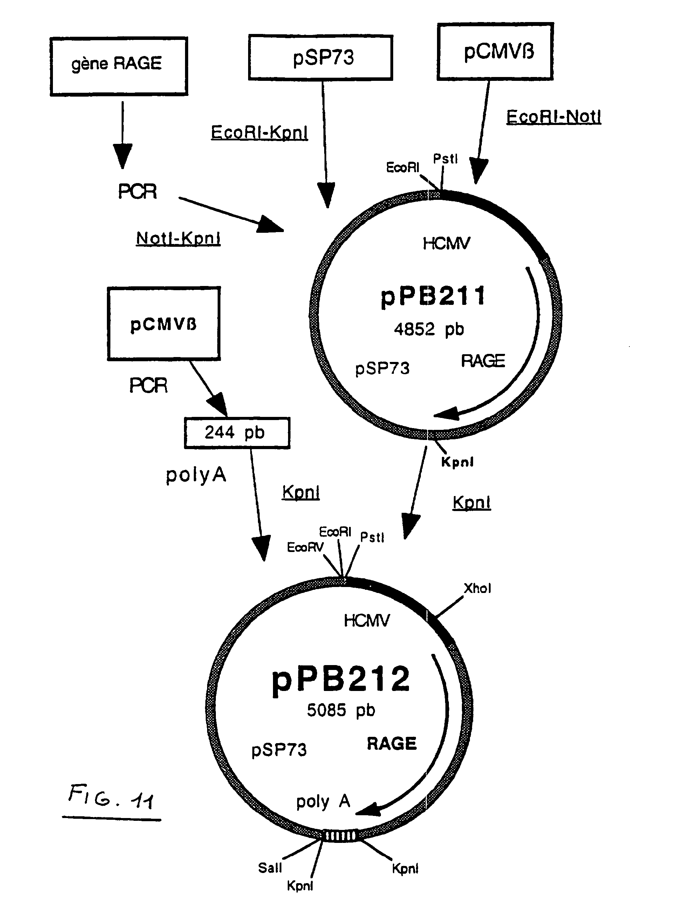

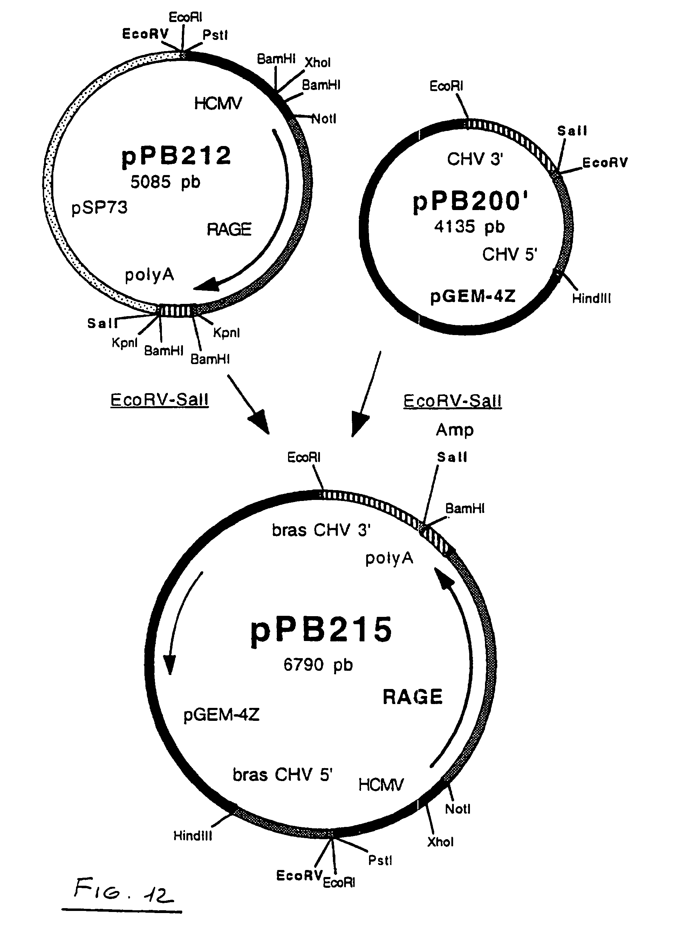

Le plasmide pCMVB (Clontech Ref. 6177-1) a été digéré par *EcoR*I et *Not*I pour isoler un fragment EcoRl-Notl de 818 pb contenant la région promotrice du gène immediate-early du cytomégalovirus humain (fragment A).
Selon les modalités techniques déjà décrites pour le virus CDV (exemple 7), l'ARN de la souche ERA du virus de la rage a été extrait et purifié à partir d'une culture de cellules Vero infectées par le virus de la rage. Une réaction RT-PCR a alors été réalisée (voir exemple 7) avec l'ARN génomique du virus de la rage (souche ERA) et avec les oligonucléotides suivants:
JCA090 (31 mer)
5'TTGCGGCCGCATGGTTCCTCAGGCTCTCCTG 3'
JCA091 (31 mer)
5'TTGGTACCTCACAGTCTGGTCTCACCCCCAC3'
pour obtenir un fragment PCR de 1597 pb contenant le gène G du virus de la rage (brevets FR-A-2 515 685 et EP-A-162 757). Ce fragment a été digéré par *Not*I et *Kpn*I pour isoler un fragment NotI-KpnI de 1586 pb (fragment B). Les fragments A et B ont été ligaturés ensemble avec le vecteur pSP73 (Promega Ref. P2221), préalablement digéré par *EcoR*I et *Kpn*I, pour donner le plasmide pPB211 (4852 pb).
Une réaction PCR a été réalisée avec le plasmide pCMVB et avec les oligonucléotides suivants:
PB088 (30 mer)
5'TTGGGTACCGCCTCGACTCTAGGCGGCCGC3'
PB089 (32 mer)
5'TTGGGTACCGGATCCGAAAAAACCTCCCACAC 3'
pour obtenir un fragment PCR de 244 pb contenant le signal de polyadénylation du gène précoce du virus SV40. Ce fragment a été digéré par *Kpn*I pour isoler un fragment Kpnl-Kpnl de 233 pb. Ce fragment a ensuite été ligaturé avec le plasmide pPB211 préalablement digéré par *Kpn*I pour donner le plasmide pPB212 (5085 pb) (Figure 11). Le plasmide pPB212 a été digéré par *EcoR*V et *Sal*I pour isoler un fragment EcoRV-Sall de 2664 pb contenant la cassette d'expression du gène G du virus de la rage. Ce fragment a été ensuite ligaturé avec le plasmide pPB200'(voir plus haut), préalablement digéré par *EcoR*V et *Sal*I, pour donner le plasmide pPB215 (6790 pb) (Figure 12).

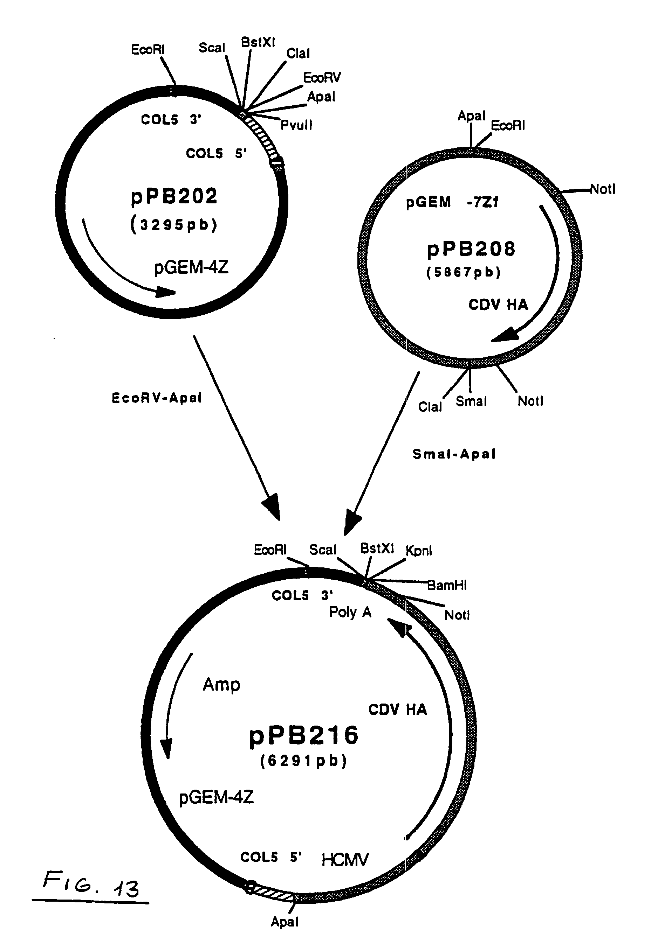

### Exemple 11: Construction du plasmide donneur pPB216 pour l'insertion de la cassette d'expression CDV HA dans le site CHV COL5 (Figure 13)

Le plasmide pPB208 (exemple 7.1.) a été digéré par *Sma*I et *Apa*I pour isoler un fragment Smal-Apal de 2909 pb contenant la cassette d'expression du gène CDV HA. Ce fragment a été ensuite ligaturé avec le plasmide pPB202 (exemple 4), préalablement digéré par *EcoRV* et *Apa*I, pour donner le plasmide pPB216 (6291 pb). Ce plasmide permet l'insertion de la cassette d'expression du gène CDV HA dans le site CHV COL5.

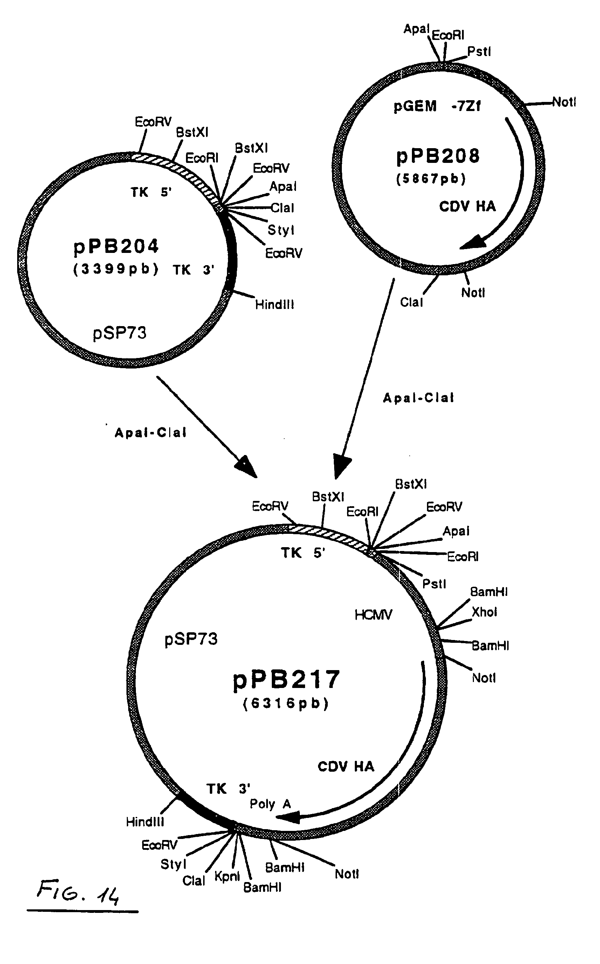

### Exemple 12: Construction du plasmide donneur pPB217 pour l'insertion de la cassette d'expression CDV HA dans le site CHV TK (Figure 14) (pas l'objet de l'invention)

Le plasmide pPB208 (exemple 7.1.) a été digéré par *Apa*I et *Cla*I pour isoler un fragment de 2920 pb contenant la cassette d'expression du gène CDV HA. Ce fragment a été ligaturé avec le plasmide pPB204 (exemple 5), préalablement digéré par *Apa*I et *Cla*I, pour donner le plasmide pPB217 (6316 pb). Ce plasmide permet l'insertion de la cassette d'expression du gène CDV HA dans le site CHV TK.

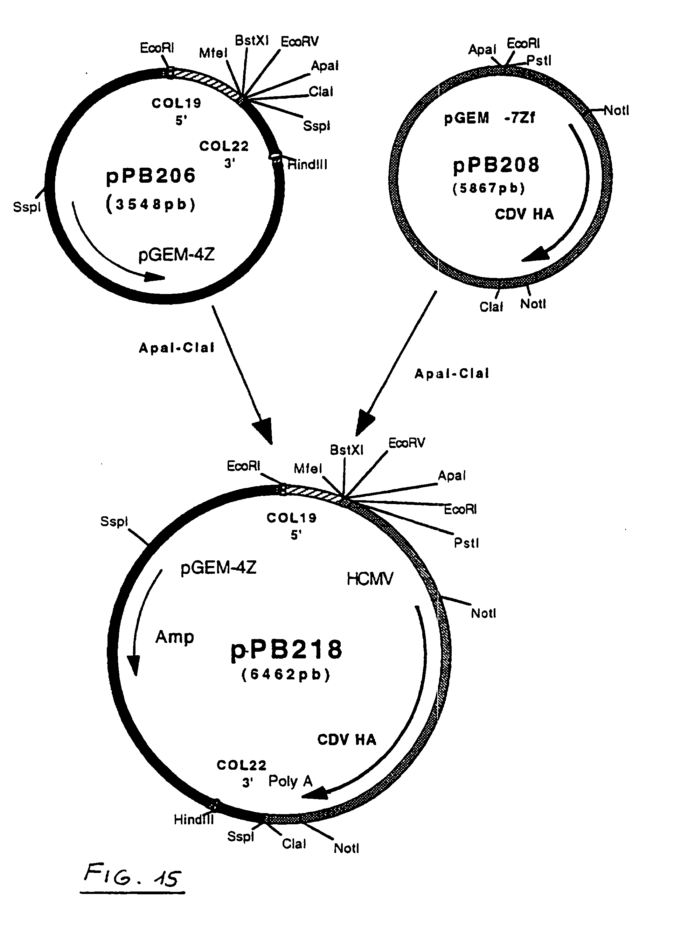

### Exemple 13: Construction du plasmide donneur pPB218 pour l'insertion de la cassette d'expression CDV HA dans le site situé entre les gènes CHV orf19 et CHV orf22 (Figure 15) (pas l'objet de l'invention)

Le plasmide pPB 208 (exemple 7.1.) a été digéré par *Apa*I et *Cla*I pour isoler un fragment de 2920 pb contenant la cassette d'expression du gène CDV HA. Ce fragment a été ligaturé avec le plasmide pPB206 (exemple 6), préalablement digéré par *Apa*I et *Cla*I, pour donner le plasmide pPB218 (6462 pb). Ce plasmide permet l'insertion de la cassette d'expression du gène CDV HA dans le site situé entre les gènes CHV orf19 et CHV orf22.

### Exemple 14: Isolement du virus recombinant vCHV01 contenant la cassette d'expression du gène CDV HA dans le site CHV COL3.

Le plasmide pPB213 (voir exemple 8) a été linéarisé par digestion avec *Hin*dIII, extrait avec un mélange phénol-chloroforme, précipité avec de l'éthanol absolu, puis repris dans de l'eau stérile.
Des cellules MDCK, formant un tapis cellulaire bien établi en boîte de Petri (Corning 4,5 cm de diamètre) ont ensuite été transfectées avec le mélange suivant:
1 µg de plasmide pPB213 linéarisé + 5 µg d'ADN viral de CHV dans 300 µl de milieu MEM et 100 µg de LipofectAMINE (Gibco-BRL Cat# 18324-012) dilués dans 300 µl de milieu (volume final du mélange = 600 µl). Ces 600 µl ont été ensuite dilués dans 3 ml (volume final) de milieu MEM et étalés sur 3.10⁶ cellules MDCK. Le mélange a été laissé en contact avec les cellules pendant 5 heures, puis éliminé et remplacé par 5 ml de milieu de culture. Les cellules ont alors été laissées en culture pendant 24 heures à + 37°C. Après 24 heures à 48 heures de culture, 1 ml de surnageant de culture a été récolté et plusieurs dilutions de ce surnageant ont été utilisées pour infecter de nouvelles cellules MDCK (cultivées en boîte de Petri (Corning 4,5 cm de diamètre) de manière à obtenir des plages isolées, chaque boîte étant infectée avec 1 ml d'une dilution du surnageant initial. Après un contact d'1 heure à 37°C, le milieu d'infection a été éliminé et remplacé par 5 ml de milieu MEM à 1 % d'agarose, maintenu en surfusion à 42°C. Lorsque l'agarose a été solidifiée, les boîtes ont été incubées 48 heures à 37°C en étuve CO₂ jusqu'à apparition de plages. La couche d'agarose a alors été éliminée et un transfert des plages virales a été réalisé sur une membrane stérile de nitrocellulose de même diamètre que la boîte de Pétri ayant servi à la culture. Cette membrane a été elle-même transférée sur une autre membrane de nitrocellulose de manière à obtenir une "copie" inversée du premier transfert. Les plages transférées sur cette dernière copie ont été alors hybridées, selon les techniques usuelles connues de l'homme de l'art, avec un fragment Notl-Notl de 1842 pb du gène CDV HA, obtenu par digestion du plasmide pPB208 (exemple 7.1), marqué à la digoxigénine (DNA Labelling Kit, Boehringer Mannheim, CAT # 1175033). Après hybridation, lavages et mise en contact avec le substrat de révélation, la membrane de nitrocellulose a été mise en contact avec un film autoradiographique. Les images d'hybridation positive sur cette membrane ont indiqué quelles étaient les plages qui contenaient des virus CHV recombinants ayant inséré la cassette CDV HA. Les plages correspondant à ces plages positives ont été découpées stérilement sur la première membrane de nitrocellulose, placées dans un tube Eppendorf contenant 0,5 ml de milieu MEM et soniquées pour libérer les virions de la membrane. Le milieu contenu dans le tube Eppendorf a été ensuite dilué en milieu MEM et les dilutions ainsi obtenues ont servi à infecter de nouvelles cultures de cellules MDCK. Un virus recombinant, contenant la cassette HCMV-IE/CDV HA/polyA insérée dans le site COL3, pur à 100 % a été ainsi isolé après 3 cycles de purification et a été appelé vCHV01. L'homologie de la recombinaison a été vérifiée par PCR en utilisant des oligonucléotides situés de part et d'autre du site d'insertion. L'absence de remaniement sur le génome du virus recombinant vCHV01, ailleurs que dans la région de recombinaison, a été vérifiée par la technique du Southern blot.

### Exemple 15: Isolement de virus CHV recombinants exprimant divers gènes étrangers

Selon les modalités décrites dans l'exemple 14, on réalise la construction de différents virus CHV recombinés en utilisant les plasmides donneurs décrits dans les exemples 9 à 13.

### Exemple 16 : Préparation de vaccins

Pour préparer un vaccin, les virus recombinants obtenus aux exemples 14 et 15 sont cultivés sur cellules MDCK. La récolte du virus recombinant se fait lorsque l'effet cytopathique est complet. Les cellules lysées et le surnageant de culture sont récoltés. Après clarification du lysat cellulaire pour éliminer les débris cellulaires, la solution virale est titrée. La solution virale est ensuite diluée dans une solution stabilisatrice pour la lyohilisation, répartie à raison d'une dose vaccinale (10² DICC50 à 10⁷ DICC50) par flacon, et enfin lyophilisée. La solution virale peut être aussi congelée si nécessaire.

### Exemple 17 : Méthodes de vaccination

Selon le mode préféré de vaccination, le vaccin obtenu selon l'invention est remis en solution ou décongelé, puis administré par voie parentérale ou mucosale, mais de préférence par voie mucosale, notamment orale et/ou nasale. La dose vaccinale sera comprise, de préférence, entre 10² DICC50 et 10⁷ DICC50. De préférence, la dose pour la voie parentérale sera comprise entre 10⁴ DICC50 et 10⁷ DICC50, et pour la voie orale et/ou nasale, entre 10² DICC50 et 10⁶ DICC50. Tel que défini, le vaccin est efficace en général après une seule administration par voie orale et/ou nasale. Cependant, des administrations répétées peuvent être nécessaires.

## Revendications

1. Virus herpès canin (CHV) comprenant et exprimant au moins une séquence nucléotidique codant pour un polypeptide antigénique d'un agent pathogène canin sélectionné dans le groupe consistant en HA du virus de la maladie de Carré, F du virus de la maladie de Carré, G du virus de la rage, VP2 du parvovirus canin, HA du virus parainfluenza de type 2, F du virus parainfluenza de type 2, OspA de *Borrelia burgdorferi* et OspB du *Borrelia burgdorferi,*
la ou les séquences nucléotidiques étant insérées dans un site non essentiel pour la réplication choisi dans le group constitué du cadre ouvert de lecture 3 et du cadre ouvert de lecture 5,
le cadre ouvert de lecture 3 correspondant dans le génome de la souche CHV F205 aux nucléotides 3040 à 4242 de la figure 1 et le cadre ouvert de lecture 5 correspondant dans le génome de la souche CHV F205 aux nucléotides 5872 à 6216 de la figure 1,
dans lequel la ou les séquences nucléotidiques sont insérées par insertion simple ou après délétion totale ou partielle.

2. Virus herpès canin (CHV) comprenant et exprimant au moins une séquence nucléotidique codant pour un polypeptide antigénique d'un agent pathogène canin, la ou les séquence nucléotidiques étant insérées dans le cadre ouvert de lecture 3, le cadre ouvert de lecture 3 correspondant dans le génome de la souche CHV F205 aux nucléotides 3040 à 4242 de la figure 1,
dans lequel la ou les séquences nucléotidiques sont insérées par insertion simple ou après délétion totale ou partielle.

3. Le virus selon la revendication 1 ou la revendication 2, dans lequel le virus est la souche CHV F205.

4. Le virus selon l'une quelconque des revendications 1 à 3, comprenant un promoteur CMV immediate-early pour exprimer la séquence insérée.

5. Le virus selon la revendication 4, dans lequel le promoteur CMV immediate-early est un promoteur CMV immediate-early murin ou humain.

6. Le virus selon l'une quelconque des revendications 1 à 5, comprenant au moins deux séquences nucléotidiques insérées dans au moins un site sous le contrôle de promoteurs eucaryotes différents.

7. Le virus selon la revendication 6, dans lequel les promoteurs eucaryotes sont des promoteurs CMV immediate-early d'origines animales différentes.

8. Le virus selon la revendication 6 ou la revendication 7, comprenant une première séquence nucléotidique associée à un promoteur CMV immediate-early et une deuxième séquence nucléotidique associée à un autre promoteur, les deux promoteurs ayant leurs extrémités 5' adjacentes.

9. Le virus selon l'une quelconque des revendications 1 à 8, comprenant en outre au moins une séquence nucléotidique codant pour un polypeptide immunomodulateur, cette séquence étant insérée dans un site non essentiel pour la réplication.

10. Le virus selon la revendication 9, dans lequel le polypeptide immunomodulateur est une cytokine.

11. Le virus selon l'une quelconque des revendications 1 à 10, comprenant, insérée dans le locus d'insertion, une cassette d'expression comprenant successivement un promoteur, deux ou plusieurs gènes séparés deux à deux par un IRES, et un signal de polyadénylation.

12. Vaccin vivant recombinant comprenant au moins un virus herpès canin selon l'une quelconque des revendications 1 à 11.

13. Formule de vaccin multivalent comprenant, en mélange ou à mélanger, au moins deux virus herpès canin selon l'une quelconque des revendications 1 à 11, ces virus CHV comprenant des séquences nucléotidiques insérées différentes.

## Claims

1. A canine herpes virus (CHV) comprising and expressing at least one nucleotide sequence encoding an antigenic polypeptide of a canine pathogenic agent selected from the group consisting of canine distemper virus HA, canine distemper virus F, rabies virus G, canine parvovirus VP2, parainfluenza virus type 2 HA, parainfluenza virus type 2 F, *Borrelia burgdoferi* OspA, and *Borrelia burgdorferi* OspB,
the nucleotide sequence(s) being inserted into a site which is non essential for replication selected from the group consisting of open reading frame 3, open reading frame 5,
open reading frame 3 corresponding within the genome of the CHV strain F205 to nucleotides 3040 to 4242 of the sequence of figure 1, and open reading frame 5 corresponding within the genome of the CHV strain F205 to nucleotides 5872 to 6216 of figure 1,
wherein the nucleotide sequence(s) is(are) inserted by simple insertion, or after total or partial deletion.

2. A canine herpes virus (CHV) comprising and expressing at least one nucleotide sequence encoding an antigenic polypeptide of a canine pathogenic agent, the nucleotide sequence(s) being inserted into the open reading frame 3,
open reading frame 3 corresponding within the genome of the CHV strain F205 to nucleotides 3040 to 4242 of the sequence of figure 1,
wherein the nucleotide sequence(s) is(are) inserted by simple insertion, or after total or partial deletion.

3. The virus according to claim 1 or claim 2, wherein the virus is the CHV strain F205.

4. The virus according to any one of claims 1 to 3, comprising a CMV immediate-early promoter to express the inserted sequence.

5. The virus according to claim 4 wherein the CMV immediate-early promoter is a murine or human CMV immediate-early promoter.

6. The virus according to any one of claims 1 to 5, comprising at least two nucleotide sequences inserted into at least one insertion site under the control of different eukaryotic promoters.

7. The virus according to claim 6, wherein the eukaryotic promoters are different CMV immediate-early promoters of different animal origin.

8. The virus according to claim 6 or claim 7, comprising a first nucleotide sequence operably linked to a CMV immediate-early promoter and a second nucleotide sequence operably linked to a second promoter; the first and second promoters being arranged so that their 5' ends are adjacent.

9. The virus according to any one of claim 1 to 8, further comprising at least one nucleotide sequence encoding an immunomodulatory polypeptide, this sequence being inserted into a site which is non essential for replication.

10. The virus according to claim 9, wherein the immunomodulatory polypeptide is a cytokine.

11. The virus according to any one of claims 1 to 10, comprising, inserted in the insertion site, an expression cassette comprising successively a promoter, two or more genes separated two by two by an internal ribosome entry site (IRES), and a polyadenylation signal.

12. A recombinant live vaccine comprising at least one canine herpesvirus according to any one of claims 1 to 11.

13. A multivalent vaccine formula comprising, as a mixture or to be admixed, at least two canine herpes viruses according to any one of claims 1 to 11, these CHV viruses comprising different inserted nucleotide sequences.

## Patentansprüche

1. Caniner Herpesvirus (CHV), welcher mindestens eine Nucleotidsequenz umfasst und exprimiert, die für ein antigenes Polypeptid eines Hundepathogens codiert, wobei das Hundepathogen ausgewählt ist aus der Gruppe bestehend aus HA des Staupevirus, F des Staupevirus, G des Rabiesvirus, VP2 des caninen Parvovirus, HA des Parainfluenzavirus Typ 2, F des Parainfluenzavirus Typ 2, OspA von Borrelia burgdorferi und OspB von Borrelia burgdorferi, wobei die Nucleotidsequenz oder die Nucleotidsequenzen an einer für die Replikation nicht essentiellen Stelle ausgewählt aus der Gruppe bestehend aus dem offenen Leserahmen 3 und dem offenen Leserahmen 5 inseriert ist/sind, wobei der offene Leserahmen 3 den Nucleotiden 3040 bis 4242 der Abbildung 1 des Genoms des Stamms CHV F205 und der offene Leserahmen 5 den Nucleotiden 5872 bis 6216 der Abbildung 1 des Genoms des Stamms CHV F205 entspricht, worin die Nucleotidsequenz oder die Nucleotidsequenzen durch einfache Insertion oder nach ganzer oder teilweiser Deletion inseriert ist/sind.

2. Caniner Herpesvirus (CHV), welcher mindestens eine Nucleotidsequenz umfasst und exprimiert, die für ein antigenes Polypeptid eines Hundepathogens codiert, wobei die Nucleotidsequenz oder die Nucleotidsequenzen in dem offenen Leserahmen 3 inseriert ist/sind, wobei der offene Leserahmen 3 den Nucleotiden 3040 bis 4242 der Abbildung 1 des Genoms des Stamms CHV F205 entspricht, worin die Nucleotidsequenz oder die Nucleotidsequenzen durch einfache Insertion oder nach ganzer oder teilweiser Deletion inseriert ist/sind.

3. Virus nach Anspruch 1 oder 2, wobei der Virus der Stamm CHV F205 ist.

4. Virus nach einem der Ansprüche 1 bis 3, welcher einen immediate-early CMV Promotor zum Exprimieren der inserierten Sequenz umfasst.

5. Virus nach Anspruch 4, wobei der immediate-early CMV Promotor ein Maus oder Mensch immediate-early CMV Promotor ist.

6. Virus nach einem der Ansprüche 1 bis 5, welcher mindestens zwei inserierte Nucleotidsequenzen an mindestens einer Stelle unter Kontrolle verschiedener eukaryotischer Promotoren umfasst.

7. Virus nach Anspruch 6, wobei die eukaryotischen Promotoren immediate-early CMV Promotoren verschiedenen tierischen Ursprungs sind.

8. Virus nach Anspruch 6 oder 7, welcher eine erste Nucleotidsequenz umfasst, die an einen immediate-early CMV Promotor assoziiert ist, und eine zweite Nucleotidsequenz, die an einen anderen Promotor assoziiert ist, wobei die 5'-Enden der beiden Promotoren aneinander grenzen.

9. Virus nach einem der Ansprüche 1 bis 8, welcher unter anderem mindestens eine Nucleotidsequenz umfasst, die für ein immunomodulierendes Polypeptid codiert, wobei diese Sequenz an einer nicht für die Replikation essentiellen Stelle inseriert ist.

10. Virus nach Anspruch 9, wobei das immunomodulierende Polypeptid ein Zytokin ist.

11. Virus nach einem der Ansprüche 1 bis 10, welcher eine in den Insertionslokus inserierte Expressionskassette umfasst, welche hintereinander einen Promotor, zwei oder mehrere jeweils durch eine IRES separierte Gene und ein Polyadenylationssignal enthält.

12. Rekombinanter Lebendimpfstoff, welcher mindestens einen caninen Herpesvirus nach einem der Ansprüche 1 bis 11 umfasst.

13. Multivalente Impfstoffformulierung, welche mindestens zwei vermischte oder zu vermischende canine Herpesviren nach einem der Ansprüche 1 bis 11 umfasst, wobei diese CHV-Viren unterschiedliche inserierte Nucleotidsequenzen umfassen.
